# EUROPEAN PATENT SPECIFICATION

(11) **EP 2 571 508 B1**
(45) Date of publication and mention of the grant of the patent: **05.10.2016**
(21) Application number: 11722149.9
(22) Date of filing: 20.05.2011
(51) Int. Cl.: A61K 35/56, A61P 9/12, A61K 35/612, A23L 33/18

(54) **PROCESS FOR PRODUCING A SHRIMP HYDROLYSATE USING ELECTRODIALYSIS**
VERFAHREN ZUR HERSTELLUNG EINES GARNELENHYDROLYSATS MITTELS ELEKTRODIALYSE
PROCÉDÉ POUR LA PRODUCTION D'UN HYDROLYSAT DE CREVETTE UTILISANT L'ÉLECTRODIALYSE

(30) Priority: 20.05.2010 GB 201008462
(43) Date of publication of application: 27.03.2013
(73) Proprietor: Marealis AS, 9008 Tromsø (NO)
(72) Inventor: RAUØ, Jaran, N-9016 Tromsø (NO)
(74) Representative: Rigby, Barbara Nicole
(86) International application number: PCT/GB2011/050954
(87) International publication number: WO 2011/144940

(56) References cited:
- FR-A1- 2 399 213
- DATABASE WPI Week 200732 Thomson Scientific, London, GB; AN 2007-330239 XP002650890, & CN 1 844 406 A (UNIV GUANGDONG OCEAN) 11 October 2006 (2006-10-11)
- DATABASE WPI Week 200624 Thomson Scientific, London, GB; AN 2006-222894 XP002650891, & CN 1 660 888 A (UNIV SHANDONG) 31 August 2005 (2005-08-31)
- HE ET AL: "High throughput and rapid screening of marine protein hydrolysates enriched in peptides with angiotensin-I-converting enzyme inhibitory activity by capillary electrophoresis", BIORESOURCE TECHNOLOGY, ELSEVIER BV, GB, vol. 98, no. 18, 31 August 2007 (2007-08-31), pages 3499-3505, XP022226415, ISSN: 0960-8524, DOI: DOI:10.1016/J.BIORTECH.2006.11.036
- CHUNLING MA ET AL: "Purification and Characterization of an Alkaline Protease from the Marine Yeast Aureobasidium pullulans for Bioactive Peptide Production from Different Sources", MARINE BIOTECHNOLOGY, SPRINGER-VERLAG, NE, vol. 9, no. 3, 7 March 2007 (2007-03-07), pages 343-351, XP019495037, ISSN: 1436-2236
- DATABASE WPI Week 199238 Thomson Scientific, London, GB; AN 1992-310684 XP002650892, & JP 4 197151 A (HACHITEI KK) 16 July 1992 (1992-07-16)

## Description

### Field of the invention

The present invention concerns a process for the production of a low salt shrimp hydrolysate, products comprising such a hydrolysate and uses of such a hydrolysate, particularly in the field of medicine and nutrition.

### Background

Hypertension is one of the risk factors for strokes, heart attacks, heart failure and arterial aneurysm, and is a leading cause of chronic renal failure. Even moderate elevation of arterial blood pressure leads to shortened life expectancy. Hypertension may be treated by targeting Angiotensin I-converting enzyme (ACE). ACE is an enzyme that participates in the body's renin-angiotensin system (RAS). It is an exopeptidase which catalyzes the conversion of the decapeptide angiotensin I to the octapeptide angiotensin II. Angiotensin II causes blood vessels to constrict, and drives blood pressure up. It also stimulates the release of aldosterone from the adrenal cortex. Aldosterone promotes sodium retention in the distal nephron, in the kidney, which also drives blood pressure up.

ACE inhibitors are used for controlling blood pressure, treating heart failure, preventing strokes, and preventing kidney damage in people with hypertension or diabetes. They also improve survival after heart attacks. Commonly used ACE inhibitors include benazepril (Lotensin), captopril (Capoten), enalapril (Vasotec), fosinopril (Monopril), lisinopril (Prinivil, Zestril), moexipril (Univasc), perindopril (Aceon), quinapril (Accupril), ramipril (Altace), and trandolapril (Mavik). These drugs often have undesired side effects and/or they are not effective enough, so there remains a need for further ACE inhibitors. There is a particular demand for natural products having ACE inhibitory effects.

The seafood industry processes a large proportion of its catch, generating waste material comprising, for example, heads, tails, shells, scales, bones, and/or skin. The processing of crustaceans typically involves freezing the catch, followed by defrosting, maturation, heating and peeling, yielding a waste product comprising heads, tails and shells.

Seafood waste comprises protein and several workers have recognised that seafood waste proteins can be enzymatically hydrolysed to yield a protein hydrolysate. The production of a protein hydrolysate typically involves dilution of the processing waste in water and addition of a protease, allowing enzymatic hydrolysis of the processing waste at a temperature at which the hydrolytic enzyme has good activity, typically 20-65° C, for a suitable length of time such as 2 hours, followed by heat inactivation of the enzyme by heating to around 80-90° C. The solids may then be separated from the aqueous phase, the aqueous phase containing the protein hydrolysate. Fats, which typically originate from cell membranes, may then be removed, for example by cooling the crude hydrolysate to cause fats to solidify, followed by removal of the solidified fats by centrifugation.

A process for the preparation of a protein hydrolysate from shrimp waste is disclosed by Gildberg and Stenberg (Process Biochemistry 2001, 36, pp 809-812). The process involves thawing shrimp waste (heads and scales), mixing with water and equilibration to about 40° C, enzymatic hydrolysis at 40° C for about 2 hours, followed by heating at 90° C for about 20 minutes, removal of solid tissues to yield a crude protein hydrolysate and purification of the crude hydrolysate by cooling to 4° C, followed by centrifugation to remove suspended solids, yielding a protein hydrolysate. These authors suggested the use of such a hydrolysate as a food or feed.

Bordenave et al. (Prep. Biochem. & Biotechnol. 32(1), 65-77, 2002) report that hydrolysates made from fish industry waste have some Angiotensin Converting Enzyme (ACE) inhibitory activity *in vitro*, although when these authors fractionated the hydrolysate of shrimp using RP-HPLC (reverse phase high performance liquid chromatography), the fractions had poor ACE inhibitory activity. He et al. (Bioresource Technology 99, 5956-5959, 2008) disclose a shrimp hydrolysate having ACE-inhibitory activity, but report that high doses of about 1200mg/kg/day were required to achieve satisfactory results.

CN1844406 and CN1660888 disclose the preparation of antihypertensive peptides from shrimp proteins.

There remains a need to develop processes for the production of a seafood protein hydrolysate and to provide hydrolysate-based products for nutritional and medical applications, particularly for the inhibition of ACE.

### Description of the Invention

The present inventors used a process according to Gildberg and Stenberg (*supra*) to prepare a shrimp protein hydrolysate. To their surprise, when they analysed the composition of the hydrolysate, they discovered that the protein hydrolysate produced using this process has a sodium chloride (NaCl) content of 14-19% w/w (weight by weight) of dry matter (see Example 1).

The processing of crustaceans, particularly shrimp and prawns, typically involves a maturation step during which the crustaceans are incubated in a mixture of water, ice and salt (NaCl) for about 24 hours to ease the peeling process. The crustaceans are then cooked and peeled, i.e. the heads, tails and shells are removed. The peeling process involves the use of large amounts of fresh water, which could be expected to remove the salt that was present during the maturation step, but sufficient salt from the maturation process apparently remains to cause the excessive and surprisingly high NaCl content of hydrolysates prepared from crustacean waste.

Other types of seafood such as fish are typically not subjected to a maturation step, but saltwater fish naturally have a NaCl content of about 1% (w/w), so dried fish have an even higher salt content (w/w dry matter). The present inventors have determined that dried hydrolysates from saltwater seafood typically have an NaCl content of at least 5% (w/w of dry matter).

Excess sodium (Na) is known to have several negative effects on the human or animal body. Most notably, it can cause hypertension and it is therefore associated with an increased risk of stroke and cardiovascular disease. Excess salt consumption has also been linked to osteoporosis, by causing calcium loss; it has also been linked to stomach cancer. NaCl can also have a negative effect on water retention and may thus cause oedema. Excess NaCl can also cause an electrolyte disturbance, i.e. disturb the natural balance of electrolytes such as sodium, chloride, potassium, calcium, magnesium and phosphate. Electrolytes regulate many vital processes including myocardial and neurological functions, fluid balance, oxygen delivery and acid-base balance, so disturbance of the natural balance of electrolytes can have serious deleterious effects on the health of an animal or human.

A high salt content, e.g. of more than 5% or more than 1%, of a protein hydrolysate is therefore clearly unacceptable for many applications, particularly for the use of the hydrolysate as a nutritional supplement or as a pharmaceutical.

The present inventors therefore set out to develop a process for reducing the NaCl content of seafood hydrolysates. Eventually, they found that electrodialysis was effective at reducing the NaCl content of seafood hydrolysates.

Thus in one aspect, the present invention provides a process for the production of a shrimp hydrolysate having angiotensin I-converting enzyme inhibitory activity comprising
(a) contacting a shrimp proteinaceous material with a protease and allowing proteolysis to take place, thereby generating a hydrolysate;
(b) increasing the dry matter content of said hydrolysate to 8-20% (w/w) through reverse osmosis;
(c) increasing the dry matter content of said hydrolysate to 40-42% (w/w) through evaporation; and then reducing the NaCl content of said hydrolysate to no more than 1% (w/w) of dry matter through electrodialysis. The electrodialysis reduces the salt content of the seafood hydrolysate.

Also disclosed is a process for removing NaCl from a seafood hydrolysate, said process comprising electrodialysis of the hydrolysate.

Electrodialysis (ED) may be used to transport salt ions from one solution through ion-exchange (semi permeable) membranes to another solution under the influence of an applied electric potential difference. A combination of anion-selective and cation-selective membranes is used. Anion-selective membranes allow the removal of CI- ions and cation-selective membranes allow the removal of Na+ ions. The solution from which it is desired to remove ions is commonly referred to as the "feed" or "diluate" and the solution into which ions are transferred is typically called "brine" or "concentrate". The concentrate is typically water, preferably deionised water. ED is carried out in an electrodialysis cell. The cell consists of a feed (diluate) compartment and a concentrate (brine) compartment formed by an anion exchange membrane and a cation exchange membrane placed between two electrodes. Typically, multiple electrodialysis cells are arranged into an electrodialysis stack, with alternating anion and cation exchange membranes forming multiple electrodialysis cells. The electrodes may be contacted with an electrode rinse, for example 1% Na₂SO₄.

During ED, the diluate stream and the concentrate stream are allowed to flow through the cell compartments. Under the influence of an electrical potential difference, the negatively charged ions such as chloride in the diluate stream migrate toward the positively charged anode. These ions pass through the positively charged anion exchange membrane, but are prevented from further migration toward the anode by the negatively charged cation exchange membrane and therefore stay in the concentrate stream, which becomes concentrated with the anions. The positively charged species such as sodium in the diluate stream migrate toward the negatively charged cathode and pass through the negatively charged cation exchange membrane. These cations also stay in the concentrate stream, prevented from further migration toward the cathode by the positively charged anion exchange membrane. The overall result of the ED process is an increase in the ion concentration in the concentrate stream and a corresponding decrease in the ion concentration in the diluate stream. As the ion concentration in the concentrate increases, e.g. when the conductivity of the concentrate increases above 20, 25, 30 or 35 mS/cm, it may be appropriate to add more deionised water to the concentrate to decrease its ion concentration, e.g. down to about 20 or 15 mS/cm.

The typical pore size of an ED membrane is about 40 Angstroms, and this determines what molecules can pass through the ED membrane. Typically, molecules of molecular weight >400 Daltons will not pass through ED membranes, whereas molecules <75 Daltons typically pass quite readily through ED membranes. The ability of molecules having a molecular weight between 75 and 400 Daltons to pass through ED membranes is affected *inter alia* by their concentration in the feed solution.

Consequently, low molecular weight molecules such as ammonia, urea, taurine and short peptides are able to cross ED membranes. The extent to which such molecules will be lost during ED depends on their concentration in the feed solution, as well as on their size, three-dimensional configuration and charge.

Many known antihypertensive compositions comprise short ACE-inhibitory peptides and there is a risk that such peptides can pass through ED membranes. Indeed, ED can, for example, result in a dramatic loss of taurine. However, surprisingly ED did not result in a loss of ACE-inhibitory activity of the hydrolysate of the present invention.

The membranes used in ED must thus allow appropriately charged ions such as CI- and Na+ respectively to pass through, whilst ideally preventing larger molecules, such as peptides, from passing through. Suitable membranes are widely available and the skilled person will know how to select suitable membranes. Examples of suitable membranes are AR103QDP (anion) and CR61 CMP (cation), both available from GE Ionics, USA.

The inventors have determined that ED may be carried out to reduce the NaCl content of the hydrolysate. Using ED, the NaCl content may be reduced by at least 10, 20, 30, 40 or 50%, more preferably by at least 60, 65, 70, 75, 80 or 85%, most preferably by at least 90, 91, 92, 93, 94, 95, 96, 97, 98 or 99%. By "reduced by 10%" is meant that the NaCl content of the hydrolysate after ED is 10% lower than the NaCl content was prior to ED. Thus, for example, if the NaCl content of the hydrolysate was 19% prior to ED, then a 10% reduction results in an NaCl content of 17.1%. In the process of the present invention, the ED is carried out until the desired reduction in NaCl content has been achieved.

Thus, the ED is carried out to yield a hydrolysate having an NaCl content of no more than 1 % w/w of dry matter, more preferably of no more than about 0.9, 0.8, 0.7, 0.6 or 0.5% w/w of dry matter, most preferably of no more than about 0.4, 0.3, 0.2, 0.1 or 0.05% NaCl (w/w of dry matter). The ED is carried out until the desired NaCl content has been reached. Methods of determining the NaCl content of a composition are well known. An example includes a titration method using silver nitrate with a potassium dichromate indicator, but a preferred example is an ion selective electrode system, which is commercially available from many suppliers including Lazar Research Laboratories, Inc. (731 N. La Brea Avenue, suite 5, Los Angeles, CA, 90038, USA)

ED will typically be carried out at room temperature, so a temperature range of between 18 and 40° C is suitable, preferably 22-35° C, e.g. 25-30° C. The hydrolysate may initially have a relatively high temperature, e.g. about 35° C, and it may then cool down for example to as little as 20° C during the ED. The pH of the hydrolysate will typically be about 8.0-9.5, preferably 8.5-9.0, e.g. 8.7-9.0, but it may be adjusted, e.g. to about 7, e.g. 6.5-7.5. Trial-scale examples of a suitable ED process are described in the Examples.

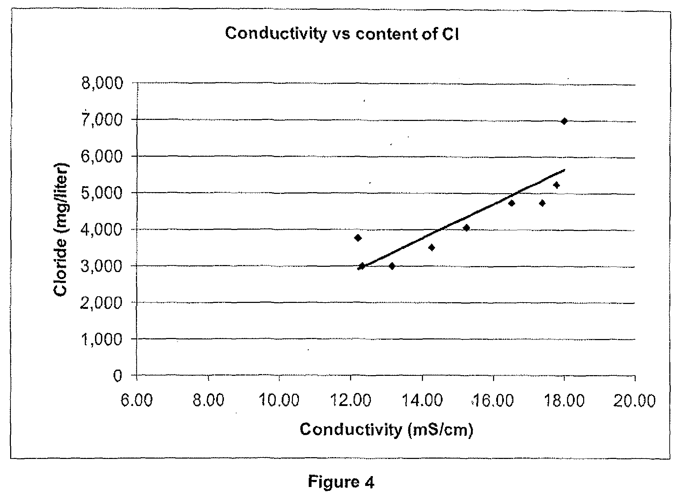

The inventors have also determined that the conductivity of the hydrolysate during ED may be taken as an indication of the NaCl content of the hydrolysate (see Example 4). Thus, the conductivity of the hydrolysate may be monitored during the ED to determine at what stage the NaCl concentration has been sufficiently lowered. For example, they have determined that at a dry matter content of 40-42%, a conductivity of about 14-17 mS/cm is indicative of a chloride concentration of about 3500 mg/litre, which corresponds to a NaCl content of 0.58%. A graph illustrating the relationship between conductivity and CI- ions in a seafood protein hydrolysate having a dry matter content of 40-42% is shown in Figure 4.

Conductivity is not solely affected by the salt concentration, it is also affected by proteins, probably due to their charge, so the desired conductivity will depend on the dry matter content of the hydrolysate. For a hydrolysate with a dry matter content of about 40-42%, the ED is preferably carried out until the conductivity is less than 30 mS/cm, more preferably less than 29, 28, 27, 26, 25, 24, 23, 22 or 21 mS/cm, most preferably about 20 mS/cm or less, e.g. about 19, 18, 17, 16 or 15 mS/cm or less. The skilled person will be able to prepare standard curves using other dry matter contents to determine what the final conductivity should be when ED is carried out using other dry matter contents.

Thus, the ED should be carried out until the desired NaCl content is achieved, suitable contents being listed above, and this may be determined by measuring the conductivity of the hydrolysate. Thus, the process may include measuring the conductivity of the hydrolysate and terminating the ED once a desired conductivity has been reached, suitable conductivity values being listed above. Conductivity may be measured using a conductivity meter, which may be obtained for example from WTW (Wissenschaftlich-Technische Werkstatten GmbH, Dr.-Karl-Slevogt-Strafte 1, D-82362 Weilheim, Germany). A suitable example is the WTW 3110 series. Alternatively, the skilled person may measure the NaCl concentration using a known method, e.g. an ion specific electrode, and terminate the ED once a desired NaCl concentration has been reached.

The skilled person will be able to determine suitable parameters such as flow rate, pressure and voltage. These parameters will vary depending on the scale of the ED. When using about 5-10 kg of hydrolysate, a flow rate of 0.5-0.6 l/min, a pressure of about 1 kg/cm and a voltage (VDC) of about 35 VDC are examples of appropriate parameters. The voltage may be reduced to about 25 VDC when the conductivity in the brine has increased significantly, e.g. to about 30 mS/cm.

Surprisingly, the NaCl removal is most effective when prior to electrodialysis, the hydrolysate is subjected to reverse osmosis and evaporation. The reverse osmosis is carried out to remove water and thereby increase the dry matter. The evaporation is also carried out to remove water and to increase the dry matter. The inventors have surprisingly found that the ED is most effective when carried out on a hydrolysate having a dry matter content of about 40-42%. They have found that a too low dry matter content (e.g. less than 30%) makes the ED less efficient, taking more time and energy to reach the desired NaCl content. When the dry matter content is too high (e.g. more than 50%), then there is a significant risk of the ED equipment becoming clogged with dry matter and covered in a protein film, reducing the effectiveness of the process. This may ultimately also damage the membranes.

Thus, the process includes a step of adjusting the dry matter content of the hydrolysate to 40-42%.

The process includes a step of reverse osmosis prior to ED. The reverse osmosis is carried out until the dry matter content of the hydrolysate is 8-20%.

Methods of carrying out reverse osmosis are known in the art. Briefly, the hydrolysate is contacted with a semi-permeable membrane and sufficient pressure is applied to force water from the hydrolysate through the membrane. The skilled person would know how to select suitable membranes. A reverse osmosis membrane must be freely permeable to water, highly impermeable to solutes, and able to withstand high operating pressures. The membrane may be cellulosic, fully aromatic polyamide or thin film composite, thin film composite being preferred. Thin film composites are made by forming a thin, dense, solute rejecting surface film on top of a porous substructure. The film may comprise or consist of aromatic polyamide, alkyl-aryl poly urea/polyamide or polyfurane cyanurate. The supporting porous sub layer may be made of polysulfone. A suitable example of a thin film composite reverse osmosis membrane is membrane AF3840C-30D available from GE Water & Process Technologies. The pressure must be sufficient to force water from the hydrolysate through the membrane, but not so high that it would damage the system. Thus, the pressure will be higher than atmospheric pressure, for example at least 102 kPa, e.g. 102 kPa - 1000 kPa, preferably about 200-500 kPa.

The process also includes a step of evaporation prior to ED. Evaporation is typically carried out in an evaporator, which removes water in the form of vapour. The solution (here, the hydrolysate) is fed into the evaporator and passes a heat source. The applied heat converts the water in the solution into vapour. The vapour is removed from the rest of the solution and is condensed while the now concentrated solution is removed. This process may be repeated one or more times.

Evaporators are commercially available, and an example of a suitable evaporator is CONVAP, S/N HC8593NCC - C2 from Alfa Laval. A suitable example of evaporation is described in Example 3. Thus, the hydrolysate may be heated to, for example, about 50-90 °C, preferably initially to about 80°C, and then lowered to about 60 °C. The hydrolysate should be left in the evaporator until the desired level of dry matter is reached, which can be calculated by measuring how much condensate the evaporator lets out from the process, and sampled by a moisture analyzer.

The dry matter content may be determined using a moisture analyzer, e.g. an infrared moisture analyzer. A suitable example is Sartorius MA35.

The evaporation is carried out until the dry matter content of the hydrolysate is 40-42%. Thus, after evaporation, the dry matter content of the hydrolysate is about 40-42%, e.g. about 41%.

The inventors have found that reverse osmosis until the dry matter content is about 13%, followed by evaporation until the dry matter content is about 40-42% is particularly efficient and advantageous.

By "prior to" in the context of a process step is meant that the step which is "prior to" a specific step chronologically precedes said step, so the prior step may be immediately prior to said specific step, i.e. without any further intermediate steps, but one or more intermediate steps may also be present. Thus. "prior to" includes in one embodiment "immediately prior to". By "immediately prior to" is meant that no intermediate steps are present between the two steps referred to and "immediately" does not in any way mean that there may not be a temporal space between the two steps. Indeed, at any stage the seafood proteinaceous waste material or the seafood hydrolysate may be stored prior to performance of the next step. Storage for short periods of time, e.g. a few minutes or hours, may conveniently be done at room temperature, e.g. 20-40° C. However, to minimise the risk of contamination and growth of microorganisms, storage may preferably be at about or below 4° C or at freezing temperature, e.g. about or below -18° C, particularly if done for long periods, e.g. more than 1 day. Storage may involve drying, if this is the case, then after storage the material should be reconstituted with a suitable solvent, preferably water, particularly deionised water to reverse the effect of the drying.

By "after" is meant that the step which is "after" a specific step chronologically follows said specific step, so the subsequent step may be immediately after said specific step, i.e. without any further intermediate steps, but one or more intermediate steps may also be present. Thus, "after" includes in one embodiment "immediately after". By "immediately after" is meant that no intermediate steps are present between the two steps referred to and "immediately" does not in any way mean that there may not be a temporal space between the two steps.

After ED, the hydrolysate may be processed further, for example it may be dried to increase the dry matter content to at least about 85, 90, 91, 92, 93, 94 or 95%, more preferably at least about 96, 97, 98, 99 or 100%. Drying may include vacuum drying, spray drying and/or freeze drying, spray drying being preferred. Lyophilizing is also preferred.

After drying, the dry material may be grinded and/or sieved in order to obtain fractions of a particular particle size range. As and when required, the dry material may be reconstituted with a suitable solvent, typically water, preferably deionised water.

Thus, the process comprises reverse osmosis and evaporation prior to electrodialysis. The reserve osmosis is prior to evaporation, which in turn is prior to electrodialysis.

Hydrolysis is a process in which an organic molecule is split into two parts by the addition of a molecule of water. Hydrolysis reactions are typically chemically or enzymatically catalysed. Chemical hydrolysis may be acid-catalysed or base-catalysed (alkaline hydrolysis, e.g. using NaOH), whereas enzymatic hydrolysis is catalysed by an enzyme.

Unless stated otherwise, any reference herein to "hydrolysis" should be taken to mean enzymatic hydrolysis. The enzymatic hydrolysis is catalysed by a protease, which catalyses hydrolysis of internal peptide bonds in proteins/polypeptides, so enzymatic hydrolysis requires the presence of a protease. Hydrolysis of a protein may also be referred to as "proteolysis", so any reference herein to "hydrolysis" of a proteinaceous material should be understood to include proteolysis. The proteolysis is enzymatic proteolysis.

The process for the production of a seafood hydrolysate will typically involve contacting seafood proteinaceous material with a protease and allowing proteolysis to take place. This step, which may be referred to as the hydrolysis step, should be carried out at a temperature at which the protease has good or optimum activity, preferably between 30 and 65° C, e.g. 35-60° C, or 45-55° C, e.g. about 50° C. The contacting may be carried out for a suitable length of time, preferably for at least 10, 20 or 30 minutes, e.g. about 1-5 hours or 1-4 hours, preferably about 2-3 hours, e.g. about 2 hours. Contacting for about 3-5 hours, e.g. about 4 hours is particularly preferred, because the inventors have determined that this results in an increase in ACE-inhibitory activity of the hydrolysate.

The step of "contacting" the proteinaceous material with a protease means that a protease is added to, or mixed with, the proteinaceous material. Thus, the process of the invention involves a step of adding a protease to the proteinaceous material. The protease may therefore be referred to as "exogenous" in that it did not form part of the proteinaceous starting material, although it is not excluded that the proteinaceous starting material contained small amounts of any endogenous proteases.

After the hydrolysis step, the process preferably includes a heating step in which the hydrolysate is heated to a temperature suitable to deactivate the protease, e.g. to around 80-95° C, e.g. about 85-90° C for about 5-30 minutes, preferably 10-20 minutes, e.g. about 15 minutes.

The hydrolysis step will typically yield a product comprising solids and liquid and after the hydrolysis the process preferably includes the step of collecting the liquid phase by separating a solid phase from a liquid phase. This may be done for example by sieving, e.g. by pressing the product against a sieve and collecting the liquid phase, or by centrifugation under conditions suitable to pellet solids whilst retaining proteins in solution, e.g. about 9000 g for 20 minutes, followed by collection of the supernatant. A decanter centrifuge, e.g. from Alfa Laval (Lund, Sweden), is particularly suitable for the removal of large particles.

The process may also comprise a step of removing fats. This may, for example, comprise cooling to cause fats to solidify, followed by removal of the solidified fats by centrifugation.

Thus, in one embodiment the process of the invention comprises steps (a) to (e) in the order listed:
(a) contacting a shrimp proteinaceous material with a protease and allowing proteolysis to take place (thereby generating a hydrolysate); (b) heating to inactivate the protease; (c) collecting the liquid phase; (d) increasing the dry matter content of said hydrolysate to 8-20% (w/w) through reverse osmosis; (e) increasing the dry matter content of said hydrolysate to 40-42% (w/w) through evaporation; and (f) reducing the NaCl content of said hydrolysate to no more than 1% (w/w) of dry matter through electrodialysis. Advantageously, said process yields a shrimp hydrolysate having a low NaCl content of no more than 1% (w/w) of dry matter. Details of these steps are discussed elsewhere herein.

In any of the embodiments disclosed herein, the shrimp is preferably *Pandalus borealis.*

It will be understood that said processes may comprise further steps. In particular, said process may also comprise a step of drying the hydrolysate, e.g. lyophilizing it.

Further additional processing steps may be carried out after ED. One or more of the following additional steps may be carried out: The hydrolysate may be centrifuged to pellet large molecules, leaving (poly)peptides in the supernatant, which may then be collected. If centrifugation is used, a low centrifugation force should be used, for example 10 000 x g.

The hydrolysate, or the supernatant obtained from centrifugation, may be filtered though a filter having a specific pore size, yielding a permeate which should only contain molecules no larger than the pore size. A pore size of about 10, 9, 8, 7, 6, 5, 4, 3, 2 or 1 kDa or less may be suitable, pore sizes of 900, 800, 700 or 600 Da or less being preferred, e.g. a pore size of about 500 Da or less. For example, filtration may be through a YC05-filter (Diaflo), and permeate should then contain molecules smaller than 500 Da. Optionally, an ultrafiltration step with a large cut-off filter may precede an ultrafiltration step with a smaller cut-off filter. For example, ultrafiltration with a 10 kDa or 5 kDa cut-off filter may be carried out and the permeate of this step may then be filtered on a 2 or 1 kDa or 500 Da cut-off filter. Filtration through a 5 kDa cut-off filter followed by a 1 kDa cut-off filter is preferred. Thus, in one embodiment, the hydrolysate is substantially free of proteins having a molecular weight of more than 500, 600, 700, 800 or 900 Da or 1, 2, 3, 4, 5, 6, 7, 8, 9, 10 kDa. Alternatively viewed, the proteinaceous components of the hydrolysate are preferably substantially all smaller than 1 kD, e.g. smaller than 500 Da.

Fractionation of the hydrolysate may be carried out. Fractionation may involve gel filtration and/or chromatography such as ion-exchange chromatography and/or high performance liquid chromatography (HPLC), e.g. reverse-phase HPLC. Preferably, ion-exchange chromatography is carried out to remove unwanted materials, followed by reverse-phase high performance liquid chromatography to separate (poly)peptides into different fractions.

Thus, in one embodiment, the process of the invention further includes one or more of the following steps (g) centrifugation; (h) filtration; (i) ion exchange chromatography; (j) HPLC.

If included in the process, each of these steps is preferably performed after ED. Most preferably, these steps, if included, are performed in the order listed (g to j).

Ion-exchange chromatography involves loading the hydrolysate (or a fraction thereof) onto an ion exchange column and eluting (poly)peptides using a buffer. An example of a suitable buffer is Na-citrate/HCl buffer, e.g. 0.02 M at pH 4. By increasing the ionic strength of the buffer in each run, each run elutes different (poly)peptides. Anion or cation exchange columns may be used. Preferably, a cation exchanger is used, for example Fractogel TSK SP 650 (Merck). The skilled person will know how to determine a suitable pH, pH 3.5 being an example. Fractionating runs should be carried out by increasing the ion strength in the buffer, for example with NaCl. The fractions obtained using ion-exchange chromatography may be tested for ACE-inhibitory activity and those fractions which have the highest ACE-inhibitory activity may be selected. Such fractions represent an ACE-inhibitory composition, which may be formulated as a pharmaceutical or nutraceutical composition and which may be used in therapy. Alternatively, such fractions may be selected for reverse-phase HPLC. As set out in the Examples, the ion-exchange chromatography fractions with pH 5.0-5.5 may be preferred.

Reverse-phase HPLC involves loading the hydrolysate (or a fraction thereof, e.g. a fraction obtained by ion-exchange chromatography, preferably the fraction with pH 5.0-5.5) onto a column made of a non-polar material and forcing a polar solvent through the column under pressure, thereby eluting (poly)peptides. Typically, the most polar (poly)peptides will be eluted prior to elution of less polar (poly)peptides. Reverse-phase HPLC may e.g. be carried out on a Shimazu instrument (LCMS - 2010 EV) equipped with a semipreparative C18 column (Inertsil ODS-3, 10 x 250 mm). An example of a suitable polar solvent is acetonitril. A gradient of the solvent, e.g. 2-98%, may be used to elute the (poly)peptides.

The fractions obtained from ion-exchange chromatography and/or HPLC may be analysed, particularly to identify fractions having ACE-inhibitory activity. Fractionation processes frequently lead to a loss in activity. This may be due to a number of factors, such as relevant polypeptides loosing their active configuration, polypeptide complexes being disrupted, co-factors being lost and the like. As described in the Examples, the inventors were able to carry out a fractionation process which yielded several fractions having good ACE-inhibitory activity. Thus, in one embodiment, the process disclosed herein includes fractionation of the hydrolysate, preferably using HPLC, most preferably RP-HPLC. The products of the fractionation are conveniently referred to herein as a "fraction of a hydrolysate", but they may also be defined as a composition obtainable by fractionation, preferably HPLC or RP-HPLC fractionation, of the hydrolysate disclosed herein. Thus, a "fraction of a hydrolysate" may be defined as a composition having ACE-inhibitory activity, said composition being obtainable by a process comprising the generation of a seafood hydrolysate as disclosed herein followed by (g) centrifugation; (h) filtration, preferably to yield a permeate containing molecules smaller than 500 Da; (i) ion exchange chromatography; and (j) HPLC of one of more of the ion-exchange chromatography fractions, preferably of the fraction with pH 5.0-5.5.

The fractions obtained via the fractionation may be tested for ACE inhibitory activity and this assay step may be a further step of the process disclosed herein. Preferably, one or more of the fractions have ACE-inhibitory activity. One or more different fractions may be combined to yield a composition preferably having ACE-inhibitory activity. A fraction may also be combined with hydrolysate which has not been fractionated, i.e. unfractionated hydrolysate, to yield a composition preferably having ACE-inhibitory activity.

By "hydrolysate" is meant the reaction product obtained subsequent to enzymatic hydrolysis of the protein components of the starting material, irrespective of whether further processing steps have been carried out after the hydrolysis step. Thus, for example the product obtained after ED or ED and filtration is also referred to as a hydrolysate. During hydrolysis, proteins are broken into smaller components including polypeptides, peptides and free amino acids. A peptide is a molecule that is formed by linking at least two amino acids via an amide bond, also called a peptide bond. Thus, a peptide comprises or consists of at least 2 amino acids. Peptides consisting of two amino acids are called dipeptides, peptides consisting of three amino acids tripeptides and so on. Peptides consisting of between 2 and 20 amino acids may be referred to as oligopeptides, and peptides having less than about 50 amino acids are typically referred to as polypeptides. The term "protein" is typically used to refer to large polypeptides or complexes of polypeptides.

The hydrolysate or fraction thereof, or more particularly its proteinaceous components, may therefore comprise or consist of proteins, polypeptides, oligopeptides, dipeptides, tripeptides, tetrapeptides, pentapeptides and free amino acids, preferably polypeptides, oligopeptides and free amino acids, most preferably oligopeptides and free amino acids. Thus, the proteinaceous components of the hydrolysate or fraction thereof are preferably predominantly oligopeptides, most preferably dipeptides, tripeptides, tetrapeptides and pentapeptides. Total amino acids may be determined as described by Pedersen et al. (Comp. Biochem. Physiol. 2003; 134B:407-16).

The free extractable amines and amino acids and total amino acids in a desalted shrimp protein hydrolysate prepared according Example 6, were determined (Example 9) and results are shown in Table 6. The hydrolysate of the present invention preferably has an amino acid composition substantially as shown in Table 6.
Other materials such as carbohydrates and/or lipids may also be present. Unless stated otherwise, any reference herein to a "hydrolysate" should be taken to be a shrimp protein hydrolysate.

By "protease" is meant an enzyme which catalyses the hydrolytic breakdown of proteins/polypeptides into polypeptides, oligopeptides and/or free amino acids. Proteases which may be used to prepare a hydrolysate may be any type of protease including endoproteases and exoproteases, endoproteases being preferred. The terms "endoprotease" refers to an enzyme that hydrolyses internal peptide bonds in oligopeptide or polypeptide chains. Suitable endoproteases include Serine proteases, Threonine proteases, Cysteine proteases, Aspartate proteases, Metalloproteases and Glutamic acid proteases, Serine proteases being preferred. Examples of suitable serine endoproteases include pepsin, trypsin, chymotrypsin and elastase. Most preferred proteases include serine proteases such as AlcalaseTM, Protamex TM, Food Grade®, Neutrase® and Novo-ProD™ (all available from Novozymes, Denmark), Protamex being especially preferred. Protamex is a Bacillus proteinase complex which is typically supplied as a powder having 1.5 AU (Anson Units) per gram. One Anson Unit is defined as the amount of enzyme which under standard conditions (i.e., 25° C., pH 7.5 and 10 min. reaction time) digests haemoglobin at an initial rate such that there is liberated per minute an amount of TCA soluble product which gives the same colour with phenol reagent as one milliequivalent of tyrosine.

Crude protease preparations may be used, e.g. crude extracts from protease-producing micro-organisms, although it is preferred to use a pure protease. The protease should preferably have at least food-grade purity.

The hydrolysate may be prepared using a single protease or two or more different proteases. When two or more different proteases are used, they may be contacted simultaneously and/or sequentially with the shrimp waste proteinaceous material. The skilled person will be able to determine how much protease should be used in the preparation of a hydrolysate. Generally speaking, higher enzyme concentrations mean a faster turnover. Typically, 0.1-1% weight of enzyme/weight proteinaceous material may be used, although higher amounts, e.g. 1.5, 2, 2.5 or 3% weight of enzyme/weight proteinaceous material may also be used.

The protease may be added in an amount of 0.01 to about 500 AU per kg proteinaceous material, preferably from about 0.1 to about 100 AU per kg, more preferably from about 0.5 to about 50 AU per kg, typically about 1, 5 or 10 AU per kg proteinaceous material.

Any reference herein to "seafood waste proteinaceous material" is meant to refer to the waste fraction generated during the processing of seafood by the seafood industry, so this fraction may *inter alia* comprise heads, bones, tails, scales, shell and/or skin. Thus, the reference to "proteinaceous" means that at least a proportion of the material is made up of protein, and it does not require all of the material to consist of protein, although this is not excluded. Shrimp waste (mainly head and scales) typically comprises about 30-35% protein (w/w of dry matter), so in one embodiment the proteinaceous material comprises about 20-45%, preferably 25-40%, or 27-37%, e.g. about 32% protein (w/w of dry matter). These values are given as w/w of dry matter, whereas the percentage of protein in w/w of the wet waste, which comprises about 80% water, is about 8%.

"Seafood" includes saltwater, estuarine and freshwater animals such as fish, shellfish and echinoderms. The seafood used in the process of the present invention is shrimp and the hydrolysate of the invention is a Pandalus borealis hydrolysate. Marine or saltwater shrimp is preferred, although in some embodiments freshwater shrimp are also preferred.

In one aspect, the present invention provides a Pandalus borealis hydrolysate having angiotensin 1-converting enzyme inhibitory activity and having an NaCl content of no more than 1% (w/w) of dry matter obtainable according to a process of the invention. Preferably, the NaCl content of the hydrolysate is no more than about 0.9, 0.8, 0.7, 0.6 or 0.5%, most preferably of no more than about 0.4, 0.3, 0.2, 0.1 or 0.05%. The seafood hydrolysate may be prepared according to the processes disclosed herein, so it may be defined as being obtainable or obtained by a process disclosed herein.

The hydrolysate prepared according to a process of the present invention, specifically according to Example 6, is preferably characterised by having an amino acid composition substantially as shown in Table 6. Preferably, over 50% of the amino acids present in the hydrolysate are present in the form of peptides of 2-10 amino acids. Preferably, only about 3% of the amino acids are present in the form of free amino acids.

The present inventors have determined that a hydrolysate prepared according to the process of the invention, specifically the process of Example 6, comprises a variety of peptides, some of which are known to have ACE-inhibitory activity (see Example 6). Some of these peptides may have low ACE-inhibitory activity on their own, but they may act synergistically with other components of the hydrolysate to inhibit ACE activity. The hydrolysate of the present invention therefore preferably comprises one or more peptides selected from peptides LF, FL, LY, LVK, NVY, KLP, LYK, LVAK, LVAH, LLTK, ALPH, LLLK and/or LNPK. These peptides consist of the sequences indicated, so the peptide LF is a dipeptide and the peptide LVK is a tripeptide and so on. More preferably, the hydrolysate comprises at least 2, 3, 4, 5, 6, 7, 8, 9, 10, 11 or 12 different peptides selected from this list, e.g. all of the peptides included in this list. Peptides NPK, LNA and/or VLAH may preferably also be present.

One or more of these peptides may be present in a hydrolysate fraction disclosed herein, e.g. a hydrolysate faction may comprise a combination of at least 2, 3, 4 or 5 peptides selected from KLP, LYK, LVAK, LVAH, LLTK and VLAH, preferably comprising all of these peptides. Also disclosed is a hydrolysate faction comprising peptide LY in combination with LNA. Also disclosed is a hydrolysate fraction comprising at least 2, preferably 3 peptides selected from LVK, NPK and LNPK.

In a further aspect, the present invention provides a pharmaceutical composition comprising a shrimp hydrolysate of the present invention and a pharmaceutically acceptable carrier or excipient.

The pharmaceutical compositions of the present invention can be formulated according to any of the conventional methods known in the art and widely described in the literature. Thus, the hydrolysate may be incorporated, optionally together with other active substances (examples of which are as described below), with one or more conventional pharmaceutically acceptable carriers, diluents and/or excipients, etc., appropriate for the particular use for a composition, to produce conventional preparations which are suitable or can be made suitable for administration. They may be formulated as liquids, as semi-solids or as solids, e.g. liquid solutions, dispersions, suspensions, tablets, pills, powders, sachets, cachets, elixirs, emulsions, syrups, and the like. The preferred form depends on the intended mode of administration and therapeutic application.

The preferred mode of administration is oral. Any physiologically compatible carrier, excipient, diluent, buffer or stabilizer can be used in the compositions of the invention. Examples of suitable carriers, excipients, diluents, buffers and stabilizers include one or more of water, saline, phosphate buffered saline, dextrose, glycerol, ethanol and the like, as well as combinations thereof. In some cases isotonic agents, e.g. sugars, polyalcohols (e.g. mannitol, sorbitol), or sodium chloride may be included. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the subject by employing procedures well known in the art.

In addition to a hydrolysate of the invention, the composition may further comprise one or more other active ingredients such as other agents which are useful for treating (pre)hypertension, examples of which include ACE inhibitors, Angiotensin II receptor blockers (ARB) drugs, beta-blockers, diuretics, calcium channel blockers, alpha-blockers, and peripheral vasodilators.

Suitable daily dosages may be readily determined, but for example 20-1500 mg hydrolysate per kg of body weight of the subject may be appropriate, 50-500 mg/kg body weight being preferred, e.g. at least 60, 70, 80, 90, 100, 120, 140, 160, 180 or 200 mg/kg body weight. A typical daily dosage will thus depend on the weight of the subject but it may for example be from 1500-20000 mg, preferably 3000-18000, 4000-15000, e.g. 8000 or 10000 mg. The Examples show that for rats, a dose of about 60 mg/kg body weight is effective. This dose may be translated into appropriate doses for humans using known methods. Particular reference is made to "Dose translation from animal to human studies revisited" by Reagan-Shaw et al. FASEB J. 2008 Mar;22(3):659-61. According to this document, a dose of 60 mg/kg body weight in rats translates into a dose of 10 mg/kg body weight in an adult human. For human subjects, a daily dose of about 5-10 mg/kg body weight may therefore be appropriate, so examples of suitable daily doses are 500-1500 mg, e.g. 600-1000 mg, or 700-800 mg. The skilled person will appreciate that suitable dosages may be achieved by administering a single dosage unit per day, or by administering two or more lower dosage units per day.

The pharmaceutical composition of the present invention may thus for example contain from about 50 mg to about 2 g of protein hydrolysate per solid dosage unit, e.g., per tablet, or a corresponding dosage in a liquid formulation.

The hydrolysate of the present invention has ACE-inhibitory activity. ACE-inhibitory activity may be assayed using known methods, for example based on the method reported by Cushman and Cheung (1971) by measuring the end product hippuric acid (HA) from the enzymatic reaction between ACE (Sigma A 6778) and the substrate HHL (Sigma H 1635). (CUSHMAN, D. W. & CHEUNG, H. S. (1971). Concentrations of Angiotensin-Converting Enzyme in Tissues of Rat. Biochimica Et Biophysica Acta 250(1), 261-265). A different suitable assay is disclosed by Vermeirssen et al. (2002), J. Biochem Biophys. Methods 51 (2002), pages 75-87. Briefly, this is a spectrophotometric method in which ACE inhibition is measured using the substrate furanacryloyl-Phe-Gly-Gly and rabbit lung acetone extract as the ACE source. The IC₅₀ may be determined.

As shown in Example 6, the inventors prepared hydrolysates having an IC₅₀ with respect to ACE inhibition of between 0.28 and 0.36 mg/ml. Thus, with respect to ACE inhibition, the IC₅₀ of the hydrolysate is preferably less than 2 or 1 mg/ml, more preferably less than 0.9, 0.8, 0.7, 0.6, 0.5, 0.4 mg/ml, most preferably less than 0.35, 0.3 or 0.25 mg/ml.

The stability of ACE inhibitory activity in the hydrolysate during storage at 20°C was also examined. After 6 months storage no reduction in inhibitory activity was detected. Thus, the hydrolysate of the present invention is preferably stable at least with respect to ACE-inhibitory activity, during storage at 20°C for at least 1, 2, 3, 4, 5 or 6 months.

Inhibition of ACE *in vivo* typically has antihypertensive effects, so the *in vivo* effect of the hydrolysate may be assayed using spontaneously hypertensive rats (SHRs). SHRs are a widely accepted animal model for hypertension and suitable strains are commercially bred. SHRs have normal blood pressure at a young age, but as they age, they develop spontaneous and long-lasting hypertension, particularly systolic blood pressure over 150 mmHg. The hydrolysate may be tested for its ability to inhibit hypertension in these rats. The inhibition may be complete, i.e. preventing the development of any hypertension, or partial, causing the hypertension to be less severe in hydrolysate-treated rats compared to untreated rats. The *in vivo* antihypertensive effects of the hydrolysate, or fraction thereof, may also be tested in other animals, e.g. using subjects suffering from hypertension or by testing the ability of the hydrolysate, or fraction thereof, to counter-act the hypertensive effects of one or more blood-pressure raising agents.

Blood pressure is generally measured in 'millimetres of mercury' (mmHg) and it is expressed in terms of systolic pressure and diastolic pressure. Systolic pressure is peak pressure in the arteries, which occurs near the end of the cardiac cycle when the ventricles are contracting. Diastolic pressure is minimum pressure in the arteries, which occurs near the beginning of the cardiac cycle when the ventricles are filled with blood. An example of normal measured values for a resting, healthy adult human is 90-120 mmHg systolic and 60-80 mmHg diastolic, e.g. 110 mmHg systolic and 70 diastolic (written as 110/70 mmHg).

For humans, "High blood pressure" is typically defined as a blood pressure that is 140/90 mmHg or above substantially each time it is taken. That is, it is "sustained" at 140/90 mmHg or above. However, "high blood pressure" also includes a high systolic pressure combined with a normal diastolic pressure, or a high diastolic pressure combined with a normal systolic pressure. Thus, "high blood pressure" or "hypertension" as used herein means a systolic pressure of 140 mmHg or above and/or a diastolic pressure of 90 mmHg or above. A subject having hypertension may thus have a systolic pressure of at least 140, 145, 150, 155, 160, 165 or 170 mmHg and/or a diastolic pressure of at least 90, 95, 100, 105, 110, 115 or 120 mmHg.

Blood pressures between 120/80 and 139/89 are referred to herein as "prehypertension", which is indicative of subjects having an increased risk of developing hypertension.

The values for normal or high blood pressure of other animals are known in the art.

Subjects having hypertension or prehypertension may benefit from receiving the hydrolysate or fraction thereof, pharmaceutical composition, or nutraceutical composition disclosed herein. In addition, subjects having (i) an existing cardiovascular disease, e.g. a subject having had a heart attack, transient ischaemic attack (TIA) or stroke, (ii) diabetes, (iii) kidney disease, and /or (iv) organ damage, particularly kidney or heart damage, may also benefit from receiving the hydrolysate or fraction thereof, pharmaceutical composition, or nutraceutical composition disclosed herein.

The hydrolysate of the present invention is preferably capable of lowering the systolic and/or diastolic blood pressure by at least 5 mmHg, preferably by at least about 6, 7, 8, 9 or 10 mmHg. In some embodiments, the hydrolysate of the present invention inhibits the systolic and/or diastolic blood pressure from increasing. This inhibition may be complete, causing the systolic and/or diastolic blood pressure to remain stable, or it may be partial, slowing down any increase in systolic and/or diastolic blood pressure. Such inhibition may be assessed for example by comparing any change in the systolic and/or diastolic blood pressure over a period prior to administration of the hydrolysate to the change over a period during administration of the hydrolysate. It may also be assessed by comparing any change in the systolic and/or diastolic blood pressure of subjects receiving the hydrolysate to control subjects who receive a placebo or no treatment.

Thus, in a further aspect there is provided a Pandalus borealis hydrolysate having angiotensin I-converting enzyme inhibitory activity and having an NaCl content of no more than 1% (w/w) of dry matter obtainable according to a process of the invention for use in therapy, preferably for use in lowering blood pressure, preventing or reducing the development or progression of prehypertension or hypertension and/or stimulating the immune response.

Any mammal may be treated, for example humans and any livestock, domestic or laboratory animal. Specific examples include mice, rats, pigs, cats, dogs, sheep, rabbits, cows and monkeys. Preferably, however, the mammal is a human.

The terms "therapy" or "treatment" as used herein include prophylactic therapy, which may result in the prevention of disease. The terms "therapy" and "treatment" include combating or cure of disease but also include the controlling, reduction or alleviation of disease or one or more of the symptoms associated therewith.

An "effective amount" as used herein can refer to a therapeutically effective amount or a prophylactically effective amount depending on the nature of the treatment. A therapeutically effective amount can be considered to be an amount necessary (at appropriate dosages and administration regimes) to achieve the desired therapeutic result. A prophylactically effective amount can be considered to be an amount necessary (at appropriate dosages and administration regimes) to achieve the desired prophylactic result. The amounts are likely to vary depending on the weight, age and sex of the patient as well as the severity of the disease.

The hydrolysate or pharmaceutical composition of the present invention has ACE-inhibitory peptides as the active ingredient, so it can be expected to have fewer side-effects than synthetic chemical ACE-inhibitors such as captopril.

In a further aspect, the present invention provides a nutraceutical composition comprising a Pandalus borealis hydrolysate having angiotensin I-converting enzyme inhibitory activity and having an NaCl content of no more than 1% (w/w) of dry matter obtainable according to a process of the invention and optionally further ingredients.

The term "nutraceutical" as used herein denotes a product that may provide health benefits in addition to nutritional benefits. The nutraceutical of the present invention has ACE-inhibitory activity. Thus, the nutraceutical may preferably lower blood pressure and/or prevent or reduce the development or progression of high blood pressure. Alternatively or in addition, it may stimulate the immune response and/or stimulate growth, particularly when administered to animals as animal feed. As shown herein, the hydrolysate of the present invention has a well-balanced amino acid composition, including a high proportion of essential amino acids, which makes it especially suitable as a nutraceutical.

The nutraceutical composition may be formulated as a supplement, e.g. it may be a solid formulation such as capsules, tablets or a powder, or a liquid formulation, such as solutions or suspensions. It may also be formulated as a food or feed for non-human animals by combining or incorporating it with one or more further edible materials. The edible materials may be liquid or solid.

Food products which may be formulated to comprise a nutraceutical composition of the present invention include but are not limited to instant powder products, food bars, meat and meat analogue products, fish and fish analogue products, breakfast cereals, dairy products, soy milk products, pasta, noodles, baked goods such as bread, pastry, cakes and biscuits, ready meals, infant formula and baby food.

Suitable examples of dairy products include cheese, ice cream, yogurt, whipping cream, sour cream and cottage cheese. Examples of meat products include, but are not limited to, processed meat and whole muscle meat. The meat analogue may be a textured vegetable or dairy protein that mimics meat in texture.

Non-limiting examples of protein fortified liquid beverages which may be formulated to comprise a nutraceutical composition according to the invention include carbonated fortified soft drinks, beer, fruit juices, fruit-flavored drinks, vegetable drinks, sports drinks, soy milk drinks, rice milk drinks, infant formula, milk, flavoured milk drinks, goat milk, liquid yogurt, buttermilk or combinations thereof.

The feed which may be formulated to comprise a nutraceutical composition according to the invention may be any animal feed, including pet food. The feed may be in a dry or moist form. Typical components of such compositions, in addition to the protein hydrolysate may include crude protein, crude fat, carbohydrates, starch, crude fibres, ash, minerals, trace elements, vitamins, fatty acids, protein and amino acids, and/or dietary fibre.

The hydrolysate of the present invention may also be formulated as a culture medium or supplement for micro-organisms.

The nutraceutical composition of the present invention may contain the hydrolysate in an amount sufficient to administer to a subject suitable dosage of the hydrolysate, suitable dosages being discussed above in the context of pharmaceutical compositions. Thus, if the nutraceutical composition is a food, the amount of protein hydrolysate contained therein is suitably in the range from about 50 mg per serving to about 1000, 2000, 3000, 4000, 5000 or 6000 mg per serving.

The nutraceutical composition of the present invention may be provided as food or feed to any animal, including terrestrial and aquatic animals. The animal may be a mammal, fish, bird, vertebrate or invertebrate, e.g. seafood, lifestock, pets and humans.

The invention will now be described in more detail in the following non-limited examples with reference to the Tables and Figures in which:
**Table 1** shows the parameters and measurements of electrodialysis of a hydrolysate having a dry matter content of 41.6% (see Example 4).
**Table 2** shows the relationship between conductivity of a hydrolysate having a dry matter content of about 40% and NaCl content of the hydrolysate once dried to achieve a dry matter content of 96%.
**Table 3** summarises the findings of optimisation of electrodialysis.
**Table 4** shows the ACE inhibitory activity, expressed in IC₅₀ values, of 4 batches of hydrolysate prepared and assayed according to Example 6.
**Table 5** shows the results of the *in vivo* effect of hydrolysate on spontaneously hypertensive rats (SHRs) (Example 8). Mean systolic blood pressures (mmHg) with standard deviations (±STDEV) of the groups. Animals were dosed with the test item (hydrolysate, items I, II, III and IV), vehicle (V; negative control) or Captopril® (VI; positive control) between study days 1-29.
**Table 6** shows the amino acid composition of a shrimp hydrolysate prepared according to Example 6 (see Example 9). Data for Atlantic cod is included for comparison.
**Table 7** shows the sequence, IC₅₀, MW and pl of peptides contained in the hydrolysate of Example 6 and the fractions of Example 7 (see Example 6).
**Figure 1** is a graph showing the change in conductivity observed during ED of a hydrolysate having a dry matter content of 12.8%. Conductivity is expressed in mS/cm, time in minutes.
**Figure 2** is a graph showing the change in conductivity observed during ED of water containing 180 g NaCl at the beginning of the process. Conductivity is expressed in mS/cm, time in minutes.
**Figure 3** is a graph showing the change in conductivity observed during ED of a hydrolysate having a dry matter content of 41.6%. Conductivity is expressed in mS/cm, time in minutes.
**Figure 4** is a graph illustrating the relationship between conductivity and Cl⁻ ions in a seafood protein hydrolysate having a dry matter content of 40-42%.
**Figure 5** is a graph showing the change in conductivity observed during ED of 3 batches of hydrolysate having a dry matter content of 40%.
**Figure 6** is a graph showing the ACE inhibitory activity of fractions of the hydrolysate (see Example 7).

### Examples

### Example 1 -Production of a shrimp hydrolysate according to prior art process

Seafood was caught off the coast of Norway. About 1000 kg of seafood waste material (heads and shells of *Pandalus borealis*) containing protein was mixed with water in a large container with a rotor.

The mixture was heated to 50 °C, and the industrial enzyme Protamex was added 1 % (w/w). The mixture was incubated at 50 °C for two hours.

After two hours, the mixture was heated to 90 °C for 10 minutes, in order to inactivate the enzymes.

The container was then emptied, and solids separated from the phase containing water, oil, minerals and protein components. This was done by a big sieve followed by a socket filter (100 micron). The aqueous phase was run through a decanter centrifuge to remove oil and larger particles.

The product was then taken to the drier in order to remove water and increase the dry matter content to >95%.

The NaCl content of the dry hydrolysate obtained using this process was determined to be >14% (w/w).

### Example 2 - Modification of process to include electrodialysis.

A hydrolysate was prepared essentially according to steps 1-6 of Example 1. Briefly, shrimp shell taken directly from the production of peeled prawns was mixed with water and hydrolysed using the industrial enzyme Protamex. The hydrolysate was passed through a decanter centrifuge (to remove larger particles).

The process was modified to include a step of electrodialysis (ED). The equipment for ED was obtained from General Electrics Water & Process Technologies (GEWPT). The equipment for electrodialysis was mounted on a skid, and contained manometers (to measure pressure), an ED-stack of anion/cation membranes and spacers, tubes, power unit with potentiometer, ammeter, voltmeter and 3 containers (approx. 8 litres each) for feed (hydrolysate), brine (deionized water) and electrode rinse (1% Na₂SO₄).

The membranes used in the stack were: Anion membrane AR103QDP and Cation membrane CR61CMP, both from GE Ionics, USA.

ED was carried out on a hydrolysate having a dry matter content of 12.8% (w/w) and a NaCl content of 1.5% (w/w). Voltage 25 VDC, temperature 27° C, flow of diluate 0.8 l/min, flow of brine 0.3 l/min, pressure about 0.9 kg/cm². After 180 minutes, ED was terminated. The resulting hydrolysate had a dry matter content of 10.4% (w/w) and a NaCl content of about 0%, whereas the final brine had a NaCl content of 1.5%. Thus, ED was found to be effective at reducing the NaCl content of the hydrolysate.

### Example 3 - Optimisation of process

The inventors determined that ED can reduce the NaCl content of the hydrolysate (see Example 2). Further steps were tested to try to optimise the process.

Reverse osmosis and/or evaporation were used to alter the dry matter content of the hydrolysate. For reverse osmosis, equipment from GEA Niro and membrane AF3840C-30D Durasan from Electrics Water & Process Technologies were used. An evaporator from Alfa Laval was used (CONVAP, S/N HC 8593NCC-C2).

ED was carried out on hydrolysate with different dry matter contents, for example 10.7%, 12.8%, 13.1%, 36.1%, 40%, 42% and 49.4%. About 4.8 kg of hydrolysate was used for each run.

Significant differences in the rates of NaCl removal were observed. For example, the rate of NaCl removal was 30 g/h for the hydrolysate having a dry matter content of 13% (w/w), but it could be increased to 68 g/h using a hydrolysate having a dry matter content of 40% (w/w).

However, a higher dry matter content, e.g. 49.4%, in the hydrolysate gave a higher risk of clogging the membranes. Consequently, a hydrolysate having a dry matter content of ∼40-42% was found to be ideal for the ED. This dry matter content can advantageously be achieved by using reverse osmosis followed by evaporation.

### Example 4 - Determination of NaCl reduction

A hydrolysate was prepared using the process of Example 1 modified as described in Examples 2 and 3.

Besides the ED equipment from GEWPT which included instruments for reading voltage, ampere, flow (litres/minute), and pressure, the following instruments were used to read the different parameters: Conductivity and temperature: WTW 3110 SET 1; pH: WTW pH330i/SET, pH electrode SenTix41;
Moisture analyzer: Sartorius MA35.

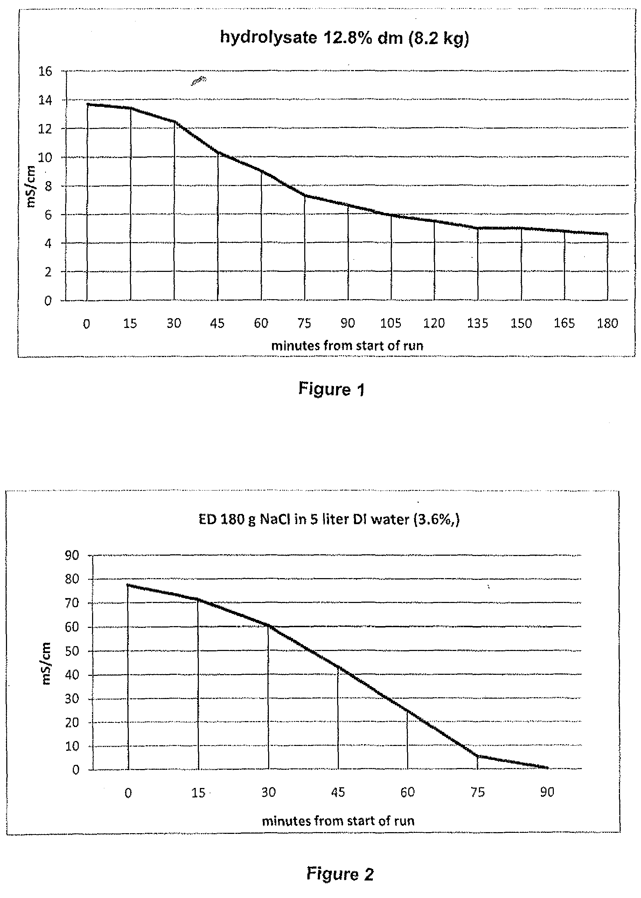

The inventors suspected that conductivity of the feed (diluate) should provide an indication of the NaCl content of the feed. However, the inventors found that the hydrolysate continues to have relatively high conductivity even after >95% of the salt is removed. This is likely due to protein components, which may carry a charge (but are too large to pass through the membranes). As can be seen from Figure 1, the conductivity may be lowered to very low levels when a hydrolysate having a low dry matter content (e.g. 12.8%) is used. This is most likely due to the low concentration of proteins in this hydrolysate.

For comparison, ED was also carried out using a solution of 180 g NaCl blended with deionised water (3.6% NaCl) as the feed to asses what the relationship between NaCl content and conductivity may be in the absence of proteins (Figure 2).

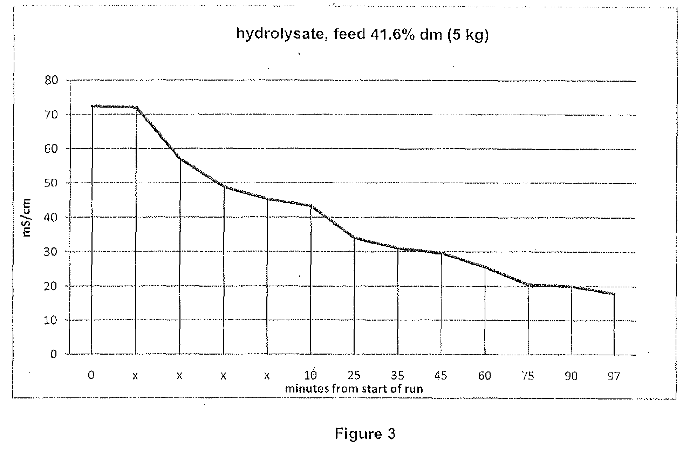

Using hydrolysates having a dry matter content of about 41%, a clear decrease in conductivity during ED was observed, as shown in Figure 3. The parameters and measurements of this experiment are shown in Table 1.

The inventors determined that in a hydrolysate having 40-42% dry matter, there is an effectively linear relationship between the concentration of CI- and the conductivity, as shown in Figure 4.

Prior to ED, a hydrolysate having a dry matter content of 41.6% had a conductivity of 72 ms/cm, and a NaCl content of 6.2 % (w/w).

After ED, the hydrolysate had a dry matter content of 38.4%, a conductivity of 17.8 ms/cm and a NaCl content of 0.7% (w/w).

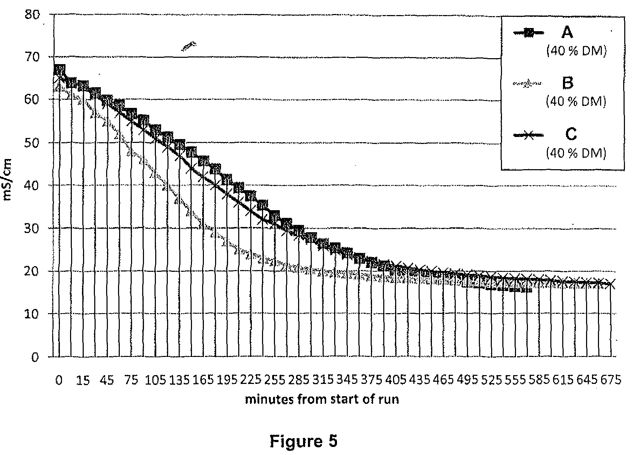

Three batches of hydrolysate were prepared according to Example 3. After ED, the hydrolysates were dried to a powder and the NaCl content was determined. The results are shown in Table 2 and in Figure 5. Thus, when carrying out ED on a hydrolysate having a dry matter content of about 40-42%, an endpoint of <20 mS/cm typically yields an NaCl content of <1%.

**Table 2**

| Batch | Analysed content of NaCl with 96% dry matter | Average mS/cm for each batch with 40% dry matter |
|---|---|---|
| A | 0.2% | 15.28 |
| B | 0.4% | 16.78 |
| C | 0.2% | 16.49 |

### Example 5 Optimisation of ED

Optimisation experiments led to the conclusion that the parameters shown in Table 3 are preferred for the electrodialysis of the hydrolysate:

**Table 3**

| | |
|---|---|
| Dry matter content of hydrolysate | ∼40-42% |
| Temperature of hydrolysate at start | ∼35°C |
| Flow rate (l/min) | 0,5-0,6 |
| Pressure (kg/cm) | ∼1 |
| Voltage (VDC) at start of process 35 VDC at the start and then reducing to 25 VDC when conductivity in brine passed 30 mS/cm) | 35 |
| Deionized (DI) water was added to the brine when conductivity in the brine reached 60 mS/cm, to maintain a conductivity of 50-60 mS/cm. | |
| When concentrate (dilute) conductivity fell to -30 mS/cm, DI water was added to the brine in order to reduce the conductivity in the brine to -25 mS/cm. At the same time the DC voltage was increased back to 35 VDC. | |
| Conductivity in the concentrate (dilute) at the end of ED process (mS/cm) | 15-20 |

The parameters for flow rate, pressure and VDC is applicable for the pilot ED-equipment and will be different for full scale process.

### Example 6 -Production of a shrimp hydrolysates having a NaCl content of about 0.1% and having ACE inhibitor activity

About 1000 kg of seafood waste material (heads and shells of *Pandalus borealis*) containing protein was mixed with water in a large container with a rotor.

The mixture was heated to 50 °C, and the industrial enzyme Protamex was added 1 % (w/w). The mixture was incubated at 50 °C for two hours.

After two hours, the mixture was heated to 90 °C for 10 minutes, in order to inactivate the enzymes.

The container was then emptied, and solids separated from the phase containing water, oil, minerals and protein components. This was done by a big sieve followed by a socket filter (100 micron).

The aqueous phase was run through a decanter centrifuge to remove oil and larger particles.

The aqueous phase was subjected to reverse osmosis, to remove water until the dry matter content has increased from approx. 4 % to 13 %. Membrane AF3840C-30D was used for reverse osmosis. Pressures of 2-4 bar (200 - 400 kPa) were used.

The retentate from the reverse osmosis process was moved over to an evaporator and more water was removed until a dry matter content of 40-42 % was reached.

The hydrolysate with 40-42 % dry matter was then subjected to electrodialysis. (Equipment obtained from General Electrics Water & Process Technologies, details as in Examples 2 and 3).

It was determined that prior to electrodialysis, the hydrolysate had a conductivity between 63-70 mS/cm, at 40-42% dry matter. Content of Cl approx. 37.500 mg/litre (equals 6,2 % NaCl and 2,44 % Na). The hydrolysate was run trough the electrodialysis stack until the conductivity was approx. 14-17 mS/cm. The content of Cl was then reduced to approx. 3.500 mg/litre (equals 0,58 % NaCl and 0,23 % Na).

The product was then taken to the drier in order to remove water and increase the dry matter content to >95%.

The ACE-inhibitory activity of the hydrolysate was measured using a method described by Vermeirssen et al. supra. By this spectrophotometric method, ACE inhibition is measured using the substrate furanacryloyl-Phe-Gly-Gly, and as ACE source rabbit lung acetone extract. 4 separate batches of hydrolysate were assayed and the results, expressed in IC50 values, were as shown in Table 4.

**Table 4**

| Batch | IC₅₀ |
|---|---|
| 1 | 0.29±0.04 mg/ml |
| 2 | 0.30±0.03 mg/ml |
| 3 | 0.28±0.01 mg/ml |
| 4 | 0.36±0.07 mg/ml |

### Example 7 - Fractionation of hydrolysate

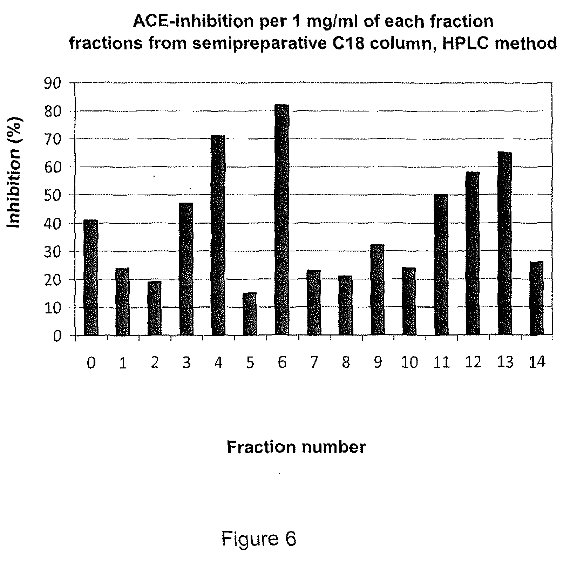

A hydrolysate was prepared according to Example 6 and fractionated as follows.
20 g of hydrolysate dissolved in 350 ml water
Dissolved hydrolysate centrifuged (10.000 x g). Majority of small peptides found in the supernatant.
Supernatant filtered through a YC05-filter (Diaflo), and permeate should contain molecules smaller than 500 Da.
Permeate fractionated in an ion exchange column (cation exchanger Fractogel TSK SP 650), pH 3.5. Fractionating runs by increasing the ion strength in the buffer with NaCl. Fractions from the ion exchange process further fractioned by RP-HPLC (semi preparative C18). Max. 20 mg per run.
Fractions from RP-HPLC analyzed for relative ACE-inhibition activity by the use of 1 mg/ml fractionated hydrolysate in 1 µg ACE-assay. ACE inhibition was assayed as described in Example 6. Results are shown in Figure 6.

The peptide composition of the RP-HPLC fractions was analysed using LCMS (liquid chromatography mass spectrometry) and individual peptides were sequenced using LC-MS/MS (liquid chromatography - tandem mass spectrometry). Peptides having the sequences identified by LC-MS/MS were then synthesized and analysed. Molecular weight and pl were calculated by ExPASy Proteomics Server. ACE-inhibitory activity was determined based on the Cushman and Cheung method mentioned elsewhere herein and expressed as the IC₅₀ (concentration that inhibits 50% of enzyme activity). Results are shown in Table 7.

### Example 8 - In vivo effect of hydrolysate

The purpose of this study was to assess the potential antihypertensive effects of the hydrolysate of the present invention. The study was carried out by examining the effects of oral administration of different dose levels (15mg/kg, 30 mg/kg, 45 mg/kg and 60 mg/kg per day) of the hydrolysate on blood pressure in spontaneously hypertensive rat (SHR).

With age, SHR rats develop spontaneous and long-lasting hypertension, particularly systolic blood pressure over 150 mmHg, and it becomes more severe in male than female animals. The normal systolic blood pressure is 116-145 mmHg in normal rats. The rats were 11-13 weeks old at the beginning of the study. 50 animals, all males, were used.

Origin of the animals: Charles River Laboratories. The animals were individually identified by tail numbering. Animals were randomly allocated to the different groups, but the animals in the positive control group were on average younger than the other animals. Fodder: RM1 E (SDS England), non-sterilised, *ad libitum*, batch 7266. Water: Tap water, *ad libitum*

Captopril® 10 mg/kg/day was used as a positive control and sterile water (vehicle) was used as a negative control.

The test item (hydrolysate) was a brownish powder in a plastic vial. The test item was stored at +2-+8°C in a sealed container and protected from light. Solutions of hydrolysate were prepared daily by mixing with sterile water to obtain the desired concentration. The test amounts of hydrolysate were administered by oral route (gavage). Oral gavage is the administration of fluids directly into the lower esophagus or stomach using a feeding needle introduced into the mouth and threaded down the esophagus. A 5ml syringe and feeding tubes were used. The dose volume was 10 ml/kg of body weight, and was based on the most recent body weight data. The animals were weighed once a week during the experiment period. The animals were examined daily and no clinical abnormalities (other than blood pressure) were observed. Administration was daily over a period of 29 days.

The systolic blood pressure (SBP) was measured on study day -4 (prior to dosing of test item) and on study days 1, 5, 7, 15, 22, 29 during the treatment period (six hours after administration of the test item), and on study days 36 and 43, i.e. during the follow up period after the treatment period had ended. SBP was measured by using a rat non-invasive tail measurement method (MLT125/R Rat Pulse Transducer and Tail Cuff, ADInstruments, Castle Hill, Australia). Prior to the measurements, the animals were warmed in a warming cabinet at 38°C for about 10 min in order to provoke detectable pulsations of the tail artery. When three consecutive blood pressure values were obtained without disturbance of the signal, the arithmetic mean was recorded as a SPB. The measurement time was limited to 10 min. During the measurements, the animals were in conscious state. If the standard deviation (STDEV) of three parallel values was more than 10, the two most nearby values were used. If there were not two similar values, all three values were used for calculation of arithmetic mean.

The results are shown in Table 5. The body weight gain was normal during the study in all groups. All values are presented as mean ± standard deviation (SD) or Standard Error of Mean (SEM), and differences are considered to be statistically significant at the P< 0.05 level

The negative control had no significant effect on SBP and the positive control lowered SBP, confirming that the experimental set up was valid.

The results of this study confirm that a dose of 60 mg/kg per day of the hydrolysate inhibited the increase of the systolic blood pressure and the impact was comparable to the effects observed with Captopril®. The trend curve of the systolic blood pressure between dose of 60 mg/kg of the test item and Captopril® 10 mg/kg are almost identical.

The mean blood pressure measurements at study day 0 (baseline) were around 170-180 mmHg in groups I-IV and VIII. In group (positive control) VI, an mean SPB values were around 160 mmHg (Table 5). The lower systolic blood pressure level in this group was probably due to younger animal age than in other groups, because blood pressure increases with age in SHRs.

**Table 5.**

| Assay | Mean systolic blood pressure (mmHg) at study days | | | | | | | | |
|---|---|---|---|---|---|---|---|---|---|
| | 0 | 1 | 5 | 7 | 15 | 22 | 29 | 36 | 43 |
| I / 15 mg/kg | 177 (±13) | 163 (±25) | 180 (±25) | 186 (±25) | 195 (±6) | 174 (±6) | 194 (±11) | 184 (±19) | 183 (±181) |
| II / 30 mg/kg | 175 (±11) | 175 (±19) | 187 (±13) | 169 (±14) | 189 (±13) | 189 (±17) | 195 (±17) | 195 (±14) | 186 (±18) |
| III / 45 mg/kg | 179 (±15) | 172 (±14) | 176 (±25) | 176 (±10) | 188 (±16) | 182 (±17) | 193 (±16) | 194 (±26) | 194 (±30) |
| IV / 60 mg/kg | 178 (±20) | 163 (±13) | 180 (±9) | 177 (±11) | 189 (±17) | 180 (±8.4) | 182 (±17) | 200 (±21) | 199 (±21) |
| V / negative control | 173 (±22) | 182 (±12) | 187 (±6) | 181 (±24) | 191 (±11) | 180 (±14) | 192 (±23) | 189 (±24) | 195 (±44) |
| VI / positive control | 162 (±16) | 159 (±7) | 165 (±19) | 151 (±14) | 168 (±16) | 161 (±18) | 165 (±11) | 177 (±9) | 178 (±17) |

### Example 9 Amino acid composition analysis

The total amino acid composition of a shrimp hydrolysate prepared according to Example 6 was analysed. Results are shown in Table 6, which includes the composition of Atlantic cod muscle protein for comparison. Whilst the amino acid composition of the shrimp hydrolysate is similar to that of Atlantic cod, there are clear differences.

The composition of essential amino acids of the shrimp hydrolysate is well balanced, indicating that it has an advantageous nutritional value.

**Table 6**

| Free extractable amines and amino acids and total amino acids in desalted shrimp protein hydrolysate as compared to total amino acids in Atlantic cod muscle protein (g/100g) | | | |
|---|---|---|---|
| Amino acid/amine | Desalted shrimp protein hydrolysate | | |
| | free | total | Cod muscle protein* |
| *Essential* | | | |
| Histidine | 0.06 | 2.60 | 2.94 |
| Isoleucine | 0.12 | 4.67 | 4.61 |
| Leucine | 0.61 | 6.85 | 8.13 |
| Lysine | 0.11 | 7.27 | 9.18 |
| Methionine | 0.14 | 2.59 | 2.96 |
| Phenylalanine | 0.34 | 4.64 | 3.90 |
| Threonine | 0.05 | 4.24 | 4.39 |
| Tryptophan | 0.04 | 1.06 | 1.12 |
| Valine | 0.12 | 5.27 | 5.15 |
| | | | |

| *Non-essential* | | | |
|---|---|---|---|
| Tyrosine | 0.13 | 3.57 | 3.38 |
| Aspartic acid | 0.02 | 8.42 | 10.24 |
| Glutamic acid | 0.10 | 11.56 | 14.92 |
| Glycine | 0.27 | 5.33 | 6.05 |
| Serine | 0.12 | 4.57 | 4.08 |
| Arginine | 0.20 | 7.13 | 5.99 |
| Alanine | 0.19 | 5.49 | 4.80 |
| Proline | 0.06 | 4.71 | 3.54 |
| Hydroxyproline | | 0.22 | |
| Cysteine/Cystine | | 0.75 | 1.07 |
| | | | |

| *Other amino acids*/*amines* | | | |
|---|---|---|---|
| Asparagine | 0.10 | | |
| Glutamine | 0.02 | | |
| 3-amino-propanoic acid | 0.02 | | |
| 4-amino-butanoic acid | 0.01 | | |
| Carnosine | 0.08 | | |
| Anserine | 0.04 | | |
| Ornithine | 0.04 | | |
| Taurine | 0.02 | | |

| | | | |
|---|---|---|---|
| * Obtained from Shahidi F, Naczk M, Pegg RB, Synowiecki J. Chemical composition and nutritional value of processing discards of cod (Gadus morhua). Food Chem 1991; 42: 145-51. | | | |

**Table 7**

| **Fraction** | **Sequence** | **IC50 µM)** | **MW (average)** | **pl (theoretical)** |
|---|---|---|---|---|
| 12 | LF | 460 | 278,35 | 5,52 |
| 13 | FL | 380 | 278,35 | 5,52 |
| 6.11 | LY | 75 | 294,35 | 5,52 |
| 0 | LVK | 225 | 358,48 | 8,75 |
| 0 | NPK* | >300 | 357,41 | 8,75 |
| 11 | NVY | >300 | 394,43 | 5,52 |
| 3 | KLP | 200 | 356,47 | 8,75 |
| 3 | LYK | 250 | 422,52 | 8,59 |
| 6.11 | LNA* | 200 | 316,36 | 5,52 |
| 3 | LVAK | >300 | 429,56 | 8,75 |
| 3 | LVAH | >300 | 438,53 | 6,74 |
| 3 | LLTK | >300 | 473,61 | 8,75 |
| 4 | ALPH | >300 | 436,51 | 6,79 |
| 9 | LLLK | >300 | 485,67 | 8,75 |
| 0 | LNPK | >300 | 470,57 | 8,75 |
| 3 | VLAH* | >300 | 438,53 | 6,71 |

## Claims

1. A process for the production of a shrimp hydrolysate having angiotensin I-converting enzyme inhibitory activity comprising
(a) contacting a shrimp proteinaceous material with a protease and allowing proteolysis to take place, thereby generating a hydrolysate;
(b) increasing the dry matter content of said hydrolysate to 8-20% (w/w) through reverse osmosis;
(c) increasing the dry matter content of said hydrolysate to 40-42% (w/w) through evaporation; and then
(d) reducing the NaCl content of said hydrolysate to no more than 1% (w/w) of dry matter through electrodialysis.

2. A process according to claim 1, wherein said shrimp is Pandalus borealis.

3. A process according to claim 1 or claim 2, wherein in step (b), the dry matter content of said seafood hydrolysate is increased to 10-15% or 10-13% (w/w).

4. A process according to any one of claims 1-3, wherein step (a) is carried out for 3-5 hours.

5. A process according to any one of claims 1-4, wherein said process further comprises fractionation of the hydrolysate using reverse phase high performance liquid chromatography (RP-HPLC).

6. A process according to claim 5, wherein said process includes steps (a) to (d) of any one of claims 1-4, centrifugation and/or filtration to yield a permeate containing molecules having a molecular size of <500 Da, ion-exchange chromatography, and then RP-HPLC.

7. A Pandalus borealis hydrolysate having angiotensin I-converting enzyme inhibitory activity and having an NaCl content of no more than 1% (w/w) of dry matter obtainable according to a process of any one of claims 2-4.

8. A pharmaceutical composition comprising a Pandalus borealis hydrolysate according to claim 7 and a pharmaceutically acceptable carrier or excipient.

9. A nutraceutical composition comprising a Pandalus borealis hydrolysate according to claim 7 and a physiologically acceptable carrier.

10. A Pandalus borealis hydrolysate according to claim 7 or pharmaceutical composition according to claim 8 for use in therapy.

11. A Pandalus borealis hydrolysate according to claim 10, for use in lowering blood pressure and/or treating or preventing the onset or progression of hypertension.

## Patentansprüche

1. Verfahren zur Herstellung eines Garnelenhydrolysats, das eine hemmende Aktivität gegen das Angiotensin I-umwandelnde Enzym aufweist, umfassend
(a) Inkontaktbringen eines proteinischen Garnelenmaterials mit einer Protease zum Ermöglichen der Proteolyse, wodurch ein Hydrolysat erzeugt wird;
(b) Erhöhen des Trockensubstanzgehalts des Hydrolysats zu 8 bis 20 Gew.-%durch Umkehrosmose;
(b) Erhöhen des Trockensubstanzgehalts des Hydrolysats zu 40 bis 42 Gew.-%durch Verdampfung; und danach
(d) Reduzieren des NaCl-Gehalts des Hydrolysats zu nicht mehr als 1 Gew.-% Trockensubstanz durch Elektrodialyse.

2. Verfahren nach Anspruch 1, wobei die Garnele Pandalus borealis ist.

3. Verfahren nach Anspruch 1 oder Anspruch 2, wobei in Schritt (b) der Trockensubstanzgehalt des Meeresfruchthydrolysats auf 10 bis 15 Gew.-% oder 10 bis 13 Gew.-% erhöht wird.

4. Verfahren nach einem der Ansprüche 1 bis 3, wobei Schritt (a) für 3 bis 5 Stunden ausgeführt wird.

5. Verfahren nach einem der Ansprüche 1 bis 4, wobei das Verfahren ferner die Fraktionierung des Hydrolysats mithilfe der Umkehrphasen-Hochleistungsflüssigkeitschromatographie (RP-HPLC) umfasst.

6. Verfahren nach Anspruch 5, wobei das Verfahren die Schritte (a) bis (d) nach einem der Ansprüche 1 bis 4, Zentrifugation und/oder Filtration zum Ergeben eines Permeats, das Moleküle enthält, die eine Molekülgröße von < 500 Da aufweisen, Ionenaustauschchromatografie und dann RP-HPLC beinhaltet.

7. Pandalus borealis-Hydrolysat mit hemmender Aktivität gegen das Angiotensin I-umwandelnde Enzym und einem NaCl-Gehalt von nicht mehr als 1 Gew.-% der Trockensubstanz, das gemäß einem Verfahren nach einem der Ansprüche 2 bis 4 erhalten werden kann.

8. Pharmazeutische Zusammensetzung, umfassend ein Pandalus borealis-Hydrolysat nach Anspruch 7 und einen pharmazeutisch verträglichen Träger oder Trägerstoff.

9. Nutrazeutische Zusammensetzung, umfassend ein Pandalus borealis-Hydrolysat nach Anspruch 7 und einen physiologisch verträglichen Träger.

10. Pandalus borealis-Hydrolysat nach Anspruch 7 oder pharmazeutische Zusammensetzung nach Anspruch 8 zur Verwendung in der Therapie.

11. Pandalus borealis-Hydrolysat nach Anspruch 10 zur Verwendung bei der Senkung von Blutdruck und/oder bei der Behandlung oder der Prävention des Einsetzens oder Fortschreitens von Bluthochdruck.

## Revendications

1. Processus pour la production d'un hydrolysat de crevette ayant une activité inhibitrice de l'enzyme de transformation d'angiotensine I comprenant
(a) la mise en contact d'une matière protéique de crevette avec une protéase et le fait de permettre à une protéolyse d'avoir lieu, produisant de ce fait un hydrolysat ;
(b) l'augmentation de la teneur en matières sèches dudit hydrolysat à 8-20 % (M/M) par osmose inverse ;
(c) l'augmentation de la teneur en matières sèches dudit hydrolysat à 40-42 % (M/M) par évaporation ; et ensuite
(d) la réduction de la teneur en NaCl dudit hydrolysat à pas plus de 1 % (M/M) de matières sèches par électrodialyse.

2. Processus selon la revendication 1, dans lequel ladite crevette est la crevette nordique (Pandalus borealis).

3. Processus selon la revendication 1 ou la revendication 2, dans lequel dans l'étape (b), la teneur en matières sèches dudit hydrolysat de fruits de mer est augmentée jusqu'à 10-15 % ou 10-13 % (M/M).

4. Processus selon l'une quelconque des revendications 1 à 3, dans lequel l'étape (a) est effectuée pendant 3 à 5 heures.

5. Processus selon l'une quelconque des revendications 1 à 4, dans lequel ledit processus comprend en outre le fractionnement de l'hydrolysat en utilisant une chromatographie liquide à haute performance en phase inverse (RP-HPLC).

6. Processus selon la revendication 5, dans lequel ledit processus inclut les étapes (a) à (d) selon l'une quelconque des revendications 1 à 4, une centrifugation et/ou une filtration pour donner un perméat contenant des molécules ayant une taille moléculaire < 500 Da, une chromatographie par échange d'ions, et ensuite une RP-HPLC.

7. Hydrolysat de crevette nordique ayant une activité inhibitrice de l'enzyme de transformation de l'angiotensine I et ayant une teneur en NaCl non supérieure à 1 % (M/M) de matières sèches que l'on peut obtenir par un processus selon l'une quelconque des revendications 2 à 4.

8. Composition pharmaceutique comprenant un hydrolysat de crevette nordique selon la revendication 7 et un vecteur ou un excipient acceptable d'un point de vue pharmaceutique.

9. Composition de nutraceutique comprenant un hydrolysat de crevette nordique selon la revendication 7 et un vecteur acceptable d'un point de vue physiologique.

10. Hydrolysat de crevette nordique selon la revendication 7 ou composition pharmaceutique selon la revendication 8 à utiliser dans une thérapie.

11. Hydrolysat de crevette nordique selon la revendication 10, à utiliser pour abaisser la tension et/ou traiter ou empêcher le début ou la progression de l'hypertension.
